# EUROPEAN PATENT APPLICATION

(11) **EP 3 730 124 A1**
(43) Date of publication of application: **28.10.2020**
(21) Application number: 18892391.6
(22) Date of filing: 04.12.2018
(51) Int. Cl.: A61K 8/34, A61K 8/37, A61K 8/92, A61Q 1/04, A61Q 19/00

(54) **SOLID COSMETIC**

(30) Priority: 18.12.2017 JP 2017241671
(71) Applicant: MITSUBISHI PENCIL COMPANY, LIMITED, Tokyo 140-8537 (JP)
(72) Inventor: NAKAJIMA Nobuyuki, Fujioka-shi, Gunma 375-8501 (JP); ENDOU Mitsuru, Fujioka-shi, Gunma 375-8501 (JP); HASEGAWA Tomoko, Fujioka-shi, Gunma 375-8501 (JP); UEHARA Junya, Fujioka-shi, Gunma 375-8501 (JP); SATOU Hiroshi, Fujioka-shi, Gunma 375-8501 (JP); HAYAKAWA Takayuki, Fujioka-shi, Gunma 375-8501 (JP)
(74) Representative: Vossius & Partner Patentanwälte Rechtsanwälte mbB
(86) International application number: PCT/JP2018/044625
(87) International publication number: WO 2019/124065

(57) **Abstract**

Provided is a solid cosmetic, although containing a large amount of glycerin to exhibit a warm feeling effect, capable of maintaining a shape-retaining property, also free from separation of a wax and the glycerin, also excellent in preservation stability over time, and suitable for lipsticks (lipstick cosmetics), massage cosmetics, and the like that can promote blood circulation by warming or massaging lips and each part of the body and provide effective lip care, skin care, and the like. The solid cosmetic contains at least 50% by mass or more of a glycerin, 1 to 15% by mass of a wax, 1 to 20% by mass of a semisolid oil, and 0.1 to 5% by mass of an ester of a polyhydroxy fatty acid and dipentaerythritol.

## Description

### Technical Field

The present invention relates to a solid cosmetic exhibiting a warm feeling effect, and more particularly relates to a solid cosmetic suitable for lipsticks (lipstick cosmetics), massage cosmetics, and the like that can promote blood circulation by warming or massaging lips and each part of the body and provide effective lip care, skin care, and the like.

### Background Art

Among cosmetics, such as facial cleansers, pack agents, and milky lotions, many cosmetics known in the art exhibit a warm feeling effect when applied to the skin of each part of the body or the skin of the whole body and provide a warm feeling and thus improve the feeling of use and the like.

Examples of components exhibiting a warm feeling effect in cosmetics known in the art include capsicum tincture, capsicum extract, vanillylnonamide (synthetic capsaicin), vanillyl butyl, and glycerin. Among components, capsicum tincture, capsicum extract, vanillylnonamide (synthetic capsaicin), and vanillyl butyl are irritating to the skin and may cause a problem when used on the face, such as the eyes or the mouth; or the skin.

In contrast, among the components exhibiting a warm feeling effect described above, glycerin is known to generate a warm feeling by heat of hydration when coming into contact with water (moisture on the body surface) and is not irritating to the skin and is highly safe. Examples of cosmetics using a glycerin known in the art include 1) a cosmetic containing a hydrated polymer, such as a starch/sodium acrylate graft polymer, and a polyol, such as 1,3-butanediol, a propylene glycol, or a glycerin, the cosmetic containing 50% by mass or more of the polyol based on the total amount of the cosmetic (see, e.g., Patent Document 1); 2) a warm feeling cosmetic containing 75 to 95% by mass of a glycerin and/or a polyethylene glycol (PEG), 0.005 to 1% by mass of a hygroscopic component, such as sericin, and a remaining amount of an additional cosmetic component (see, e.g., Patent Document 2) ; and 3) a warm feeling gel cosmetic containing 15 to 25% by mass of a polyethylene glycol with an average degree of polymerisation 4 to 30 and 60 to 85% by mass of a glycerin (see, e.g., Patent Document 3).

However, each cosmetic exhibiting a warm feeling effect of a glycerin or the like in Patent Documents 1 to 3 is not solid but liquid or gel, and a large amount of glycerin, if present in the formulation, causes a problem of unable to maintain the solidity. Thus, the current state is that the advent of a solid cosmetic maintaining the solidity of a lipstick or the like and also exhibiting a warm feeling effect has been longed for.

On the other hand, many solid lip sticks (lipstick cosmetics) containing a glycerin, a wax, and the like are also known, and examples known in the art include a lipstick containing a colourant; a wax, such as ceresin or candelilla wax; and a glycerin (see, e.g., Patent Documents 4 and 5).

However, the solid lip sticks containing a glycerin, a wax, and the like of Patent Documents 4 and 5 contain only a small amount of glycerin or the like, for example, 2% by mass and 3% by mass, respectively, and thus cannot exhibit a warm feeling effect. Thus, in the current state, a solid cosmetic maintaining the solidity while containing a large amount of glycerin to exhibit a warm feeling effect is unknown.

### Prior Art Documents

### Patent Documents

Patent Document 1: Japanese Patent Application Laid-Open No. 2003-300826 A (Claims and Examples 1 to 10)
Patent Document 2: Japanese Patent Application Laid-Open No. 2010-100577 A (Claims and Example 1)
Patent Document 3: Japanese Patent Application Laid-Open No. 2011-116726 A (Claims and Examples 1 to 7)
Patent Document 4: Japanese Patent Application Laid-Open No. Hei. 10-167951 A (Claims and Example 9)
Patent Document 5: Japanese Patent Application Laid-Open No. 2001-294515 A (Claims and Examples 20 and 21)

### Summary of Invention

### Problems to be solved by the Invention

In light of the problems of the related art, the current state, and the like described above, the present invention is to solve problems, and an object of the present invention is to provide a solid cosmetic exhibiting a warm feeling effect, in particular, a solid cosmetic suitable for lipsticks (lipstick cosmetics), massage cosmetics, and the like that can promote blood circulation by warming or massaging lips and each part of the body and provide effective lip care, skin care, and the like.

### Means to solve Problems

As a result of diligent research in light of the current state and the like of the related art, the present inventors found that a formulation containing at least a large amount of glycerin to exhibit a warm feeling effect, and also a wax, a semisolid oil, and a specific component each in a specific range can provide an intended solid cosmetic, and thus completed the present invention.

That is, the solid cosmetic according to the present invention contains at least 50% by mass or more of a glycerin, 1 to 15% by mass of a wax, 1 to 20% by mass of a semisolid oil, and 0.1 to 5% by mass of an ester of a polyhydroxy fatty acid and dipentaerythritol.

The melting point of the wax is preferably 60°C or higher.

The ester of a polyhydroxy fatty acid and dipentaerythritol is preferably dipentaerythrityl tri-polyhydroxystearate.

Furthermore, the solid cosmetic preferably contains a moisturiser.

### Effects of the Invention

The present invention provides a solid cosmetic, although containing a large amount of glycerin to exhibit a warm feeling effect, capable of maintaining the solidity, also free from separation of a wax and the glycerin, also excellent in preservation stability over time, and suitable for lipsticks (lipstick cosmetics), massage cosmetics, and the like that can promote blood circulation by warming or massaging lips and each part of the body and provide effective lip care, skin care, and the like.

### Description of Embodiments

Hereinafter, embodiments of the present invention will be described in detail.

A solid cosmetic according to the present invention contains at least 50% by mass or more of a glycerin, 1 to 15% by mass of a wax, 1 to 20% by mass of a semisolid oil, and 0.1 to 5% by mass of an ester of a polyhydroxy fatty acid and dipentaerythritol.

The glycerin used in the present invention is any glycerin used as a component to exhibit a warm feeling effect and used as a cosmetic, and not particularly limited, and various types of glycerin can be used.

The content of the glycerin is preferably 50% by mass or more (hereinafter merely abbreviated as "%"), more preferably 70% or more, and particularly preferably 75% or more based on the total amount of the solid cosmetic in terms of exhibiting a warm feeling effect and the like. On the other hand, the content of the glycerin is desirably 95% or less in terms of adjusting the other components, maintaining the solidity, and the like.

The solid cosmetic with the content of the glycerin of less than 50% would not sufficiently exhibit a warm feeling effect, and thus is not preferred.

Examples of the wax used in the present invention include ceresin wax, candelilla wax, solid paraffin wax, microcrystalline wax, Fischer-Tropsch wax, carnauba wax, beeswax, Japanese wax, spermaceti wax, polyethylene wax, polypropylene wax, montan wax, ozokerite wax, rice bran wax, and silicone wax. One of waxes may be used alone or two or more may be used in combination.

Among waxes, a wax with a melting point of 60°C or higher and more preferably with a melting point of 60 to 100°C is preferably used in terms of improving fluidity during filling and achieving even better melting during use and moisturising feeling in the skin after application.

Examples of the wax with a melting point of 60°C or higher include ceresin wax, candelilla wax, beeswax, paraffin wax, microcrystalline wax, rice bran wax, castor hydrogenated oil, carnauba wax, and ceresin wax.

The content of the wax is 1 to 15%, more preferably 3 to 15%, and particularly preferably 5 to 15% based on the total amount of the solid cosmetic in terms of maintaining the solidity, blocking moisture, and the like.

The cosmetic with the content of the wax of less than 1% would not be solid and incapable of blocking moisture into the cosmetic, and on the other hand, the cosmetic with the content of the wax of more than 15% would give a stiff feeling of use and be uncomfortable to use.

The semisolid oil used in the present invention is any oil in a semisolid state at ordinary temperature (25°C) and commonly used in cosmetics and not particularly limited, and a hydrocarbon semisolid oil, an ester semisolid oil, an ether semisolid oil, a silicone semisolid oil, or the like can be used.

Specifically, examples of the semisolid oil include hydrocarbon semisolid oils such as vaseline; ester semisolid oils, such as dipentaerythrite fatty acid esters such as dipentaerythrityl hexa(hydroxystearate/stearate/rosinate), dipentaerythrityl (hydroxystearate/stearate/rosinate), dipentaerythrityl hexahydroxystearate, dipentaerythrityl tetra(hydroxystearate/isostearate), and dipentaerythrityl (hydroxystearate/isostearate), hydrogenated castor oil fatty acid esters such as hydrogenated castor oil stearate, hydrogenated castor oil isostearate, and hydrogenated castor oil hydroxystearate, cholesterol fatty acid esters such as cholesteryl hydroxystearate, phytosterol fatty acid esters such as phytosteryl oleate and phytosteryl macadamia nut oil fatty acid, and hydrogenated vegetable oils such as a hydrogenated coconut oil and a hydrogenated palm oil, ester semisolid oils such as (phytosteryl/isosteryl/cetyl/stearyl/behenyl) dimer dilinoleate, and macadamia nut oil polyglyceryl-6 esters behenate; ether semisolid oils such as hydroxyalkyl dimer dilinoleyl ether; and silicone semisolid oils such as dimethicone cross polymers and (dimethicone/vinyl dimethicone) cross polymers. One or two or more of semisolid oils can be used.

Among semisolid oils, vaseline, macadamia nut oil polyglyceryl-6 esters behenate, and an ester semisolid oil are desirably used in terms of improving fluidity during filling and achieving excellent melting during use and moisturising feeling in the skin after application.

The content of the semisolid oil is 1 to 20%, more preferably 1 to 15%, and particularly preferably 5 to 15% based on the total amount of the solid cosmetic in terms of retaining the glycerin in the solid cosmetic and compatibility with the wax.

The solid cosmetic with the content of the semisolid oil of less than 1% would not sufficiently retain the glycerin and not sufficiently retain moisture in the skin after application, and on the other hand, the solid cosmetic with the content of the semisolid oil of more than 20% would give a sticky feeling and fail to give a sufficiently good feeling of use.

The ester of dipentaerythritol and a polyhydroxy fatty acid used in the present invention is a component imparting solidity to the solid cosmetic although the solid cosmetic contains a large amount (50% or more) of glycerin, and in addition, the ester of dipentaerythritol and a polyhydroxy fatty acid has excellent dispersibility and improved compatibility with the other components.

Dipentaerythritol has six hydroxyl groups to be esterified, and thus dipentaerythritol and a polyhydroxy fatty acid ester can range from a monoester to a hexaester, which is a full ester, depending on the degree of esterification and can be obtained by reacting dipentaerythritol and a polyhydroxy fatty acid in a suitable molar ratio by a well-known esterification method.

In the present invention, a triester is preferably used.

In addition, the polyhydroxy fatty acid is a polymer of fatty acids having at least one hydroxyl group in the molecule, and examples of such fatty acids having a hydroxyl group include linear or branched, saturated or unsaturated hydroxy fatty acids having 12 to 22 carbon atoms, such as hydroxylauric acid, hydroxymyristic acid, hydroxypalmitic acid, hydroxystearic acid, and hydroxybehenic acid. Among polyhydroxy fatty acids, polyhydroxy fatty acids having 16 to 20 carbon atoms are preferred, and in particular, 12-hydroxystearic acid having 18 carbon atoms is preferred.

The ester of dipentaerythritol and a hydroxy fatty acid used in the present invention is preferably dipentaerythrityl tri-polyhydroxy C₁₂₋₂₂ fatty acids and more preferably dipentaerythrityl tri-polyhydroxy C₁₆₋₂₀ fatty acids. Specifically, examples include dipentaerythrityl tri-polyhydroxystearate, dipentaerythrityl tri-polyhydroxylaurate, dipentaerythrityl tri-polyhydroxymyristate, and dipentaerythrityl tri-polyhydroxypalmitate. One of esters may be used alone or two or more may be used in combination.

Among the dipentaerythrityl tri-polyhydroxy fatty acids described above, dipentaerythrityl tri-polyhydroxystearate is preferably used in the present invention, and examples of commercially available products include "SALACOS WO-6" manufactured by Nisshin OilliO Group, Ltd.

The content of the ester of dipentaerythritol and a hydroxy fatty acid according to the present invention is preferably 0.1 to 5% and more preferably 0.1 to 3% based on the total amount of the solid cosmetic in terms of retaining the glycerin and maintaining dispersibility of the glycerin.

The solid cosmetic with the content of the ester of dipentaerythritol and a hydroxy fatty acid of less than 0.1% would be unable to maintain the solidity and would cause outflow of the glycerin or permeation of water vapor into the cosmetic, and on the other hand, the solid cosmetic with the content of the ester of dipentaerythritol and a hydroxy fatty acid of more than 5% would impair the feeling of use.

Furthermore, in the present invention, the solid cosmetic preferably contains a moisturiser as a component to incorporate moisture after application and enhance the warm feeling of the glycerin.

Examples of the moisturiser that can be used include components derived from plants (e.g., lavender, glasswort, and cogongrass); honey; propylene glycol and butylene glycol (such as 1,3-butylene glycol); amino acids and derivatives thereof such as alanine, serine, leucine, isoleucine, threonine, glycine, proline, hydroxyproline, glucosamine, theanine, natural moisturisers (NMT), and SA amino acids; proteins, peptides, or hydrolysates thereof such as collagen and elastin; sugar alcohols such as sorbitol; phospholipids such as lecithin and hydrogenated lecithin; mucopolysaccharides such as hyaluronic acid, sodium hyaluronate, acetylhyaluronic acid, sodium acetylalted-hyaluronate, heparin, and chondroitin; NMF-derived components such as lactic acid, sodium pyrrolidone carboxylate, and urea; polyglutamic acid; polymers having a phospholipid polar group such as MPC polymers; polyoxypropylene methyl glucosides; trimethylglycine (betaine); hydroxyethylurea; acrylate/acrylamide/dimethyl diallyl ammonium chloride copolymers; pyrrolidone carboxylic acid; and sorbitol.

Among moisturisers, a component incorporating moisture and increasing moisturisation is desirably used, such as honey; amino acids and derivatives thereof, such as alanine, serine, leucine, isoleucine, threonine, glycine, proline, hydroxyproline, glucosamine, theanine, aspartic acid, valine, arginine, glutamic acid, natural moisturisers (NMT), and SA amino acids; and propylene glycol and butylene glycol (such as 1,3-butylene glycol).

The content of the moisturiser is preferably from 0.1 to 10% and more preferably 1 to 10% based on the total amount of the solid cosmetic in terms of incorporating moisture after application to further enhance the warm feeling of the glycerin and further increasing the maintenance of moisturisation after application to the skin.

The solid cosmetic with the content of the moisturiser of less than 0.1% would not further enhance the warm feeling of the glycerin, and on the other hand, the solid cosmetic with the content of the moisturiser of more than 10% would give a sticky feeling of use.

Examples of an additional optional component that can be used in the solid cosmetic according to the present invention within a range that does not impair the effect of the present invention include colourants, such as pigments; powders; fibres; surfactants; ultraviolet absorbers; vitamins; anti-fading agents; skin-whitening components; antioxidative components; anti-aging components; keratin-softening components; cell activation components; antioxidants; antifoamer; cosmetic components; preservatives; perfumes; and refreshing agents.

Furthermore, in the present invention, in addition to the glycerin, the solid cosmetic may contain as a component having a warm feeling effect an appropriate amount of a non-irritating component used in cosmetics, for example, ethylene glycol, diethylene glycol, hexylene glycol, polyethylene glycol, dipropylene glycol, polypropylene glycol, isoprene glycol, diglycerin, triglycerin, tetraglycerin, hexaglycerin, decaglycerin, and trimethylpropanol. One of components may be used alone or two or more may be used in combination.

Furthermore, in the present invention, the glycerin generates a warm feeling by heat of hydration when coming into contact with water (moisture on the body surface), and thus the glycerin if hydrated with moisture in the formulation from the beginning would not generate heat. Thus, the solid cosmetic is required to be produced without addition of water (including captured water from the components contained and captured water from the atmosphere), and the moisture content in the solid cosmetic is preferably as low as possible and more preferably zero (0) or 1% or less.

The applications of the solid cosmetic according to the present invention can be applied to make-up cosmetics, such as lipsticks, foundations, and cheek rouges; skin care cosmetics, such as eye zone sticks, skin-whitening sticks, wrinkle concealer sticks, and roughness concealer sticks; massage cosmetics or pack cosmetics applied to the skin of each part such as the face, the neck, the arms, the hands, the states, the chest, the abdomen, the lower half of body, the buttocks, the legs, and the feet or the skin for the whole body skin; and the like.

In addition, the form of the solid cosmetic according to the present invention may be any of a stick form, a cake form, a sheet form, a polyhedron form, a powder form, or the like.

A method for producing the solid cosmetic according to the present invention is not particularly limited, but examples include a method of dry molding using a filling machine with a screw feeder, a box filling machine, or the like; and a wet molding method of filling and molding using a volatile oil and then removing the volatile oil to mold. For example, the solid cosmetic can be obtained by adding a glycerin under stirring a semi-solid oil and an ester of dipentaerythritol and a hydroxy fatty acid to solidify the glycerin, then adding a wax, filling the mixture into a container, such as a jar container, a metal dish, or a stick container, and cooling and solidifying the mixture.

The solid cosmetic according to the present invention is, in particular, preferably a stick type used by feeding. Here, the shape of the stick type container is not particularly limited, varying from an elongated thin shape to a flat shape, and can be spherical or hemispherical, as necessary.

Although the solid cosmetic according to the present invention thus configured contains a large amount of glycerin to exhibit a warm feeling effect, the glycerin is held by the ester of dipentaerythritol and hydroxy fatty acid and further enveloped by the hydrophobic semisolid oil, thus, a high concentration of glycerin is further encapsulated, and the solidity can be maintained in synergy with the wax. The solid cosmetic is also free from separation of the wax component and the glycerin, and the wax blocks moisture in the atmosphere, and thus the solid cosmetic can be stored in a stick container or the like and thus is excellent in preservation stability over time. The present invention thus provides the solid cosmetic suitable for lipsticks (lipstick cosmetics), massage cosmetics, and the like that can promote blood circulation by warming or massaging lips and each part of the body and provide effective lip care, skin care, and the like. In particular, in lipstick cosmetics, the solid cosmetic is optimum for lip care in, such as promoting blood circulation with a warm feeling effect, providing moisture to the lips, and adding plump feeling and gloss to the lips.

### Examples

Next, the present invention will be described in further detail with reference to examples and comparative examples, but the present invention is not limited to the following examples and the like.

### [Examples 1 to 9 and Comparative Examples 1 to 8]

Each solid cosmetic (ϕ1 × 5 cm) was prepared according to the formulation shown in Table 1 below by stirring under warming until the mixture became homogeneous and then cooling. Each solid cosmetic obtained in Examples 1 to 9 and Comparative Example 1 to 8 was tested for feeling of use, warm feeling, preservation property, and low temperature applicability by each test method described below.
Each solid cosmetic used in the test was filled into a feeding container and used.

These results are shown in Table 1 below.

### [Test method]

### (1) Feeling of use

Feeling when each resulting solid cosmetic was applied to the skin was subjected to a sensory evaluation according to the following evaluation criteria.

### Evaluation criteria:

o: The solid cosmetic can be applied smoothly without resistance.
Δ: The solid cosmetic is applied with some resistance or collapses.
×: The solid cosmetic cannot be applied or cannot maintain the solidity.

### (2) Warm feeling

Each resulting solid cosmetic was applied to the skin, and then whether warm feeling can be sensed was subjected to a sensory evaluation according to the following evaluation criteria.

### Evaluation criteria:

⊚: Warm feeling is clearly sensed
○: Warm feeling is sensed.
Δ: Warm feeling is slightly sensed.
×: Warm feeling is not sensed.

### (3) Preservation property

Each resulting solid cosmetic was evaluated for separation of a wax and a glycerin in preservation over time according to the following evaluation criteria.

### Evaluation criteria:

o: Not separated under room temperature (25°C, the same applies hereinafter) after one month.
Δ: Separated liquid slightly comes out at room temperature within one month.
×: Separated liquid significantly comes out within one month at room temperature, or a wax and a glycerin separates from the beginning.

### (4) Low temperature applicability

Each resulting solid cosmetic was applied to the lips of a person who stayed in an environment of 5°C for 10 minutes and subjected to a sensory evaluation according to the following evaluation criteria.

### Evaluation criteria:

○: The solid cosmetic can be applied without problem, and warm feeling is significantly sensed in an environment of 5°C.
Δ: The solid cosmetic can be applied, and warm feeling is slightly sensed in an environment of 5°C.
×: The solid cosmetic cannot be applied, and no warm feeling is sensed in an environment of 5°C.

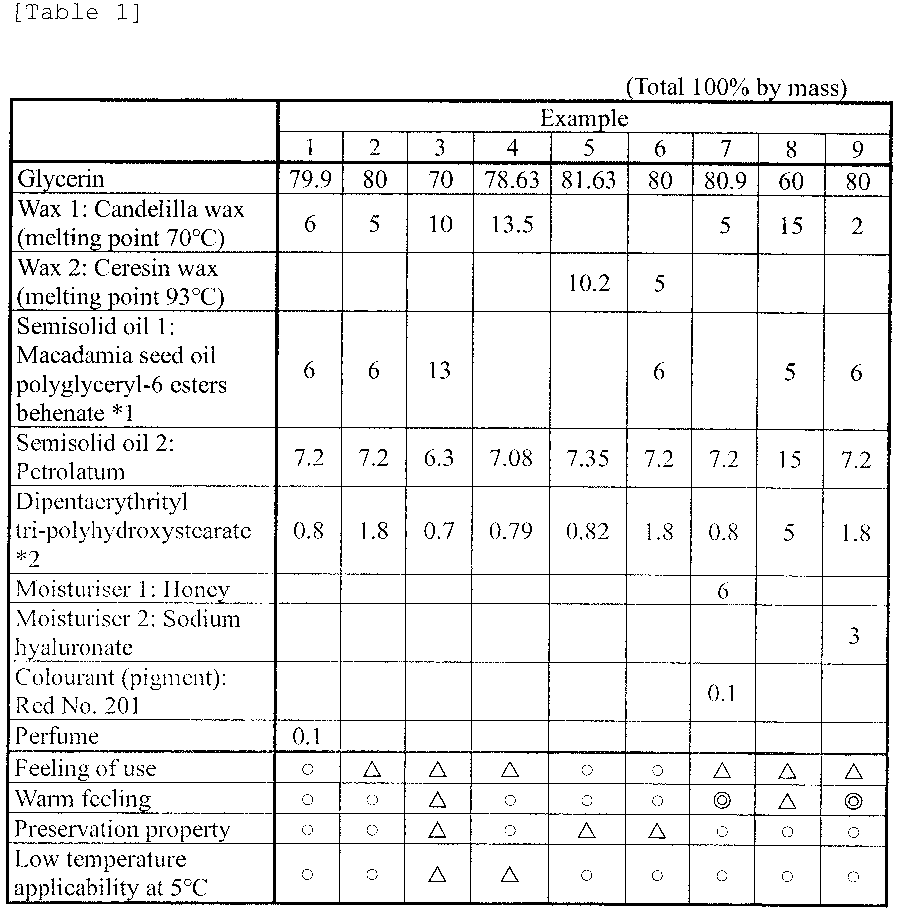

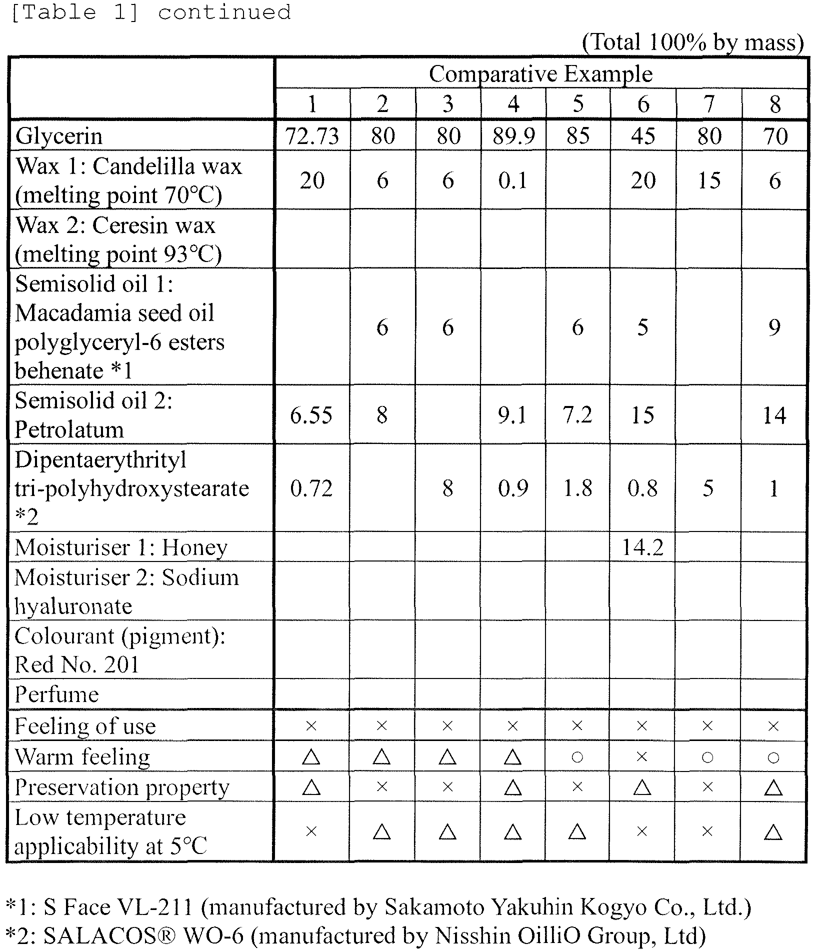

As is clear from the results in Table 1 above, in comparison with Comparative Examples 1 to 8, which were out of the scope of the present invention, Examples 1 to 9 according to the present invention were confirmed to be excellent in all of the feeling of use, warm feeling, preservation property, and low temperature applicability.

In contrast, as seen from Comparative Examples individually, Comparative Examples 1, 4, and 5 had the wax content out of the scope of the present invention (1 to 15%), Comparative Examples 2 and 3 had a content of the ester of a polyhydroxy fatty acid and dipentaerythritol out of the scope of the present invention (1 to 5%), Comparative Example 6 contained only a small amount of glycerin (45%), and Comparative Examples 7 and 8 had a content of the semisolid oil out of the scope of the present invention (1 to 20%). Such preparations turned out to be incapable of maintaining the effect of the present invention.

### Industrial Applicability

The solid cosmetic is obtained which is suitable for lipsticks (lipstick cosmetics), massage cosmetics, and the like that can promote blood circulation by warming or massaging lips and each part of the body and provide effective lip care, skin care, and the like.

## Claims

1. A solid cosmetic **characterised by** containing at least:
50% by mass or more of a glycerin,
1 to 15% by mass of a wax,
1 to 20% by mass of a semisolid oil,
and 0.1 to 5% by mass of an ester of a polyhydroxy fatty acid and dipentaerythritol.

2. The solid cosmetic described in claim 1, wherein a melting point of the wax is 60°C or higher.

3. The solid cosmetic described in claim 1 or 2, wherein the ester of a polyhydroxy fatty acid and dipentaerythritol is dipentaerythrityl tri-polyhydroxystearate.

4. The solid cosmetic described in any one of claims 1 to 3, further containing a moisturiser.
